Europäisches Patentamt

European Patent Office

Office européen des brevets

(19)

(11) Publication number : **0 138 835**
**B1**

(12)
# EUROPEAN PATENT SPECIFICATION

(45) Date of publication of patent specification :
09.09.87

(51) Int. Cl.⁴ : **A 61 B 10/00**

(21) Application number : **84900720.8**

(22) Date of filing : **07.02.84**

(86) International application number :
**PCT/DK 84/00011**

(87) International publication number :
**WO/8403034 (16.08.84 Gazette 84/20)**

(54) STAND-OFF CELL FOR AN ULTRASONIC SCANNER HEAD.

(30) Priority : **07.02.83 DK 511/83**

(43) Date of publication of application :
**02.05.85 Bulletin 85/18**

(45) Publication of the grant of the patent :
**09.09.87 Bulletin 87/37**

(84) Designated contracting states :
**AT BE CH DE FR GB LI LU NL SE**

(56) References cited :
DE-B- 2 906 474
US-A- 4 346 717

(73) Proprietor : **Medical Innovation Company A/S**
**Tornerosevej 127**
**DK-2730 Herlev (DK)**

(72) Inventor : **Drue, Herbert Renald Christian**
**Jens Ulrichs Allé 26**
**DK-5250 Odense SV (DK)**

(74) Representative : **Ryrién, Evert et al**
**ALFONS HEDBERGS PATENTBYRÄ AB Aschebergs-**
**gatan 35**
**S-411 33 Göteborg (SE)**

EP 0 138 835 B1

Jouve, 18, rue St-Denis, 75001 Paris, France

## Description

The invention relates to a stand-off cell for an ultrasonic scanner head, which stand-off cell is removably mounted on the scanner head by means of an attachment face and which is in contact with the patient through a contact surface and which furthermore has a number of free surfaces.

In medical diagnostics it is often desired to make a so-called biopsy, i. e. by means of a needle a sample is taken from the patient. The position of the needle is often critical and for this reason it is important to be able to determine the needle position exactly. This applies for example when taking samples of the amniotic fluid.

Attempts have been made to supervise the needle position by means of ultrasonic scanning wherein emitted ultrasonic impulses are reflected from the needle and its surroundings and are used for making images i. e. by means of a microcomputer and a cathode ray tube.

Ordinary scanner heads consist of a row of transducer elements placed in alignment. One example is the scanner head which is described in the US Patent Specification No. 4 346 717. Such scanner heads have the drawback, when used for biopsy, that they do not allow supervision of the first 20-50 mm of the area into which the needle is introduced. This is due to the fact that the needle first has to be lead diagonally in under the scanner head before it enters the scanner head supervision area.

In order to eliminate this drawback it is known for example from the German Patent Specification No. 2 906 474 to use special biopsy heads. These consist of an ordinary linear scanner head through which a channel has been made for the introduction of the needle. However, in order to make room for this channel it is necessary to remove a number of transducer elements in the centre of the head. This creates an area that the scanner head is unable to supervise and this will often be inconvenient when introducing the needle. These special biopsy heads furthermore have the drawback that they are very expensive. They even cost more than a normal scanner head.

When one wishes to supervise ultrasonically an area which lies close to the surface of the body, it is furthermore known for example from the UK Patent Application No. 2 009 563 to use a spacer unit between the scanner itself and the body.

The aim of the invention is to disclose a stand-off cell which may be mechanically mounted on a conventional scanner head and be acoustically connected to the emitting surface of the scanner head in a removable manner. The stand-off cell is contactable with the patient through a contact surface and is also formed with a number of free surfaces. Owing to the arrangement of the invention it becomes possible to supervise the whole course of the needle inside the patient during the biopsy sample taking. This is achieved in accordance with the invention in that the stand-off cell has a slit for engaging a biopsy needle or a hypodermic needle and that the slit extends between that part of the contact surface in the field of view of the scanner head and one of its free surfaces, the stand-off cell being made of a material with substantially the same acoustic impedance as that of the patient's tissue.

As the stand-off cell is made of a material having almost the same acoustic impedance as tissue it will be possible to register the whole course of the needle « through » this. Due to the slit, the needle will first penetrate into the patient in a place which lies within the supervision area of the scanner head. The position of the needle in a slit provides the further advantage that the scanner head with the stand-off cell can be removed from the needle without pulling the needle out of the patient. This makes it possible to perform biopsy in an easier manner just as it makes it possible to utilize the scanner head better. This is now only used while the needle itself is being placed in position. The stand-off cell can easily be made of a suitable plastics material, and therefore it will be very cheap. Thus, it becomes possible to be free to dispose of a suitable number of stand-off cells which makes it possible to use any scanner head optimum for many different purposes.

A stand-off cell according to the invention may be characterized in that the contact surface is parallel with the attachment face of stand-off cell to the scanner head. For many purposes this is suitable and very simple embodiment of a stand-off cell.

By providing the stand-off cell with one or more reflection surfaces, as defined in claim 3, it becomes possible to make stand-off cells wherein the path of the ultrasonic wave is « broken » inside the stand-off cell. This may be convenient when the biopsy has to be performed in places that are not easily accessible.

As defined in claim 4 it may be a characteristic feature of the stand-off cell according to the invention that the contact surface extends at right angles to the attachment face. When the ultrasonic waves are being emitted horizontally against a reflection surface, which forms an angle of 45° to the contact surface, a deflection of 90° of the ultrasonic waves is obtained. In this manner it becomes possible to introduce a needle into the centre of an ultrasonic field at a large number of different angles, and for example also at right angles to the contact surface.

By mounting a guide means in front of the needle, as defined in claim 5, good control of this is obtained.

Owing to the arrangement of the releasable and adjustable guide means referred to in claim 6 it becomes possible easily to place the needle at well-defined angles relative to the contact surface, and also to remove the stand-off cell in a simple manner from the needle.

As mentioned in claim 7, it may be expedient to make the slit in a needle-guide member which is secured to the stand-off cell so that it can be disconnected, as the scanner head 6 itself with the stand-off cell that can easily be removed from the needle and the needle-guide member.

The invention will be described in closer detail with reference to the drawing, wherein

Figure 1 shows in a sectional view a scanner head with a stand-off cell according to the invention,

Figure 2 shows a stand-off cell according to the invention with guide means for the needle,

Figure 3 shows a second embodiment of a stand-off cell according to the invention,

Figure 4 shows another embodiment of the invention,

Figure 5 shows an embodiment of the invention, wherein the scanner head forms an angle of 45° with the contact surface,

Figure 6 shows another embodiment of the invention, and

Figure 7 shows a needle-guide member as seen in a sectional view along the line VII-VII of Fig. 6.

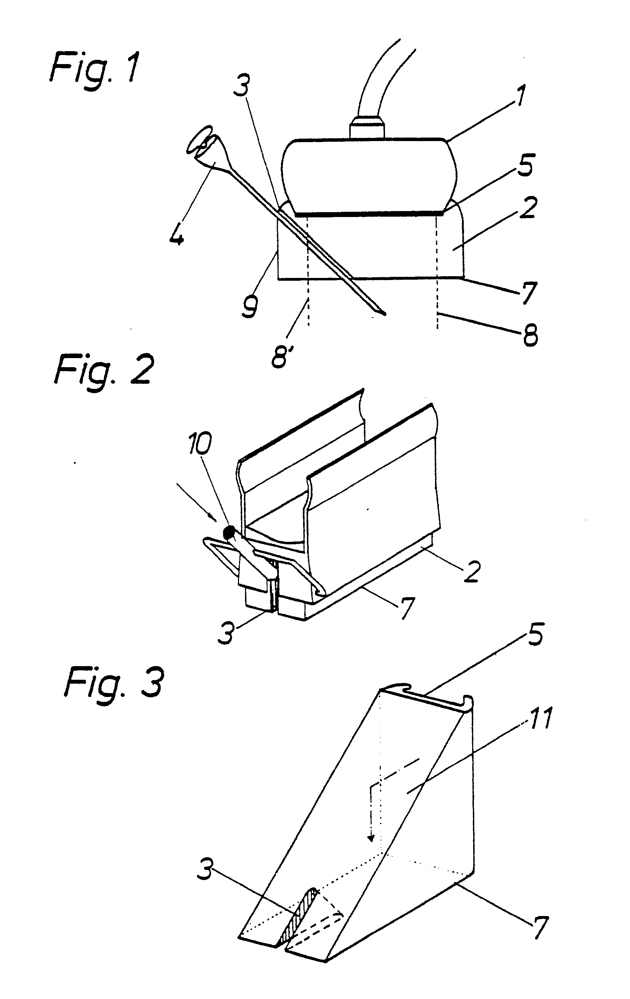

Fig. 1 shows an ordinary scanner head 1. To the head there is attached a stand-off cell 2 according to the invention at the attachment face 5. Between the stand-off cell 2 and the scanner head 1 a thin layer of a suitable paste has been applied to ensure good acoustic interconnection between the parts. The same paste preferably is used between the stand-off cell 2 and the patient.

The stand-off cell 2 is made of a plastics material having an acoustic impedance which essentially is the same as that of the patient's tissue. The plastics material furthermore must have a poor dampening effect on the ultrasonic waves. It has been found that the dampening effect of elastic materials on the ultrasonic waves often is too large, and for this reason the stand-off cell can be made of a possibly liquid-filled plastics material. In this way efficient transmission of acoustic energy is achieved without inconvenient reflections at the transition between the surfaces.

The biopsy needle 4 is lead through the slit 3 which is formed between the free side face 9 and the contact surface 7. As can be seen from Fig. 1, the needle first enters the patient in the supervision area of the scanner head which is defined by the lines 8 and 8' in Fig. 1. The slit 3 also supports the needle 4 during the introduction thereof into the patient. When the needle 4 is fully introduced the scanner head 1 and the stand-off cell 2 may be removed without further measures as the needle can be pulled through the slit.

The shape of the stand-off cell can be very simple, and consequently it may be produced very cheaply, e. g. by machining processes. Moreover, it will be simple to produce stand-off cells for any conceivable scanner head.

Fig. 2 shows how a stand-off cell 2 according to the invention can be fitted with needle-guide means 10. These could consist e. g. of a tube with a strap for supporting the needle. Owing to this arrangement the biopsy needle is safely guided. The guide means 10 can be set at various angles to the contact surface, and can of course be made in many other ways than the one shown here. When the needle-guide means 10 are connected to the stand-off cell 2 in such a manner that they can be released, the stand-off cell will be free for other uses without removal of the biopsy needle from the patient.

Fig. 3 shows a stand-off cell wherein the contact surface 7 and the attachment face 5 extend at right angles to each other.

With different configurations of the slit 3 a large range of variations can be obtained as concerns the angle of the needle relative to the patient. Since the acoustic impedance in air differs considerably from the acoustic impedance in the stand-off cell, an area which is directed horizontally against the attachment face 5 will be deflected 90° by the reflection surface 11. The ultrasonic area will thus be directed vertically down into the patient and it becomes possible to lead the needle vertically down in the centre of the sound area. This gives very precise and efficient control of the needle, while at the same time this embodiment is very material saving.

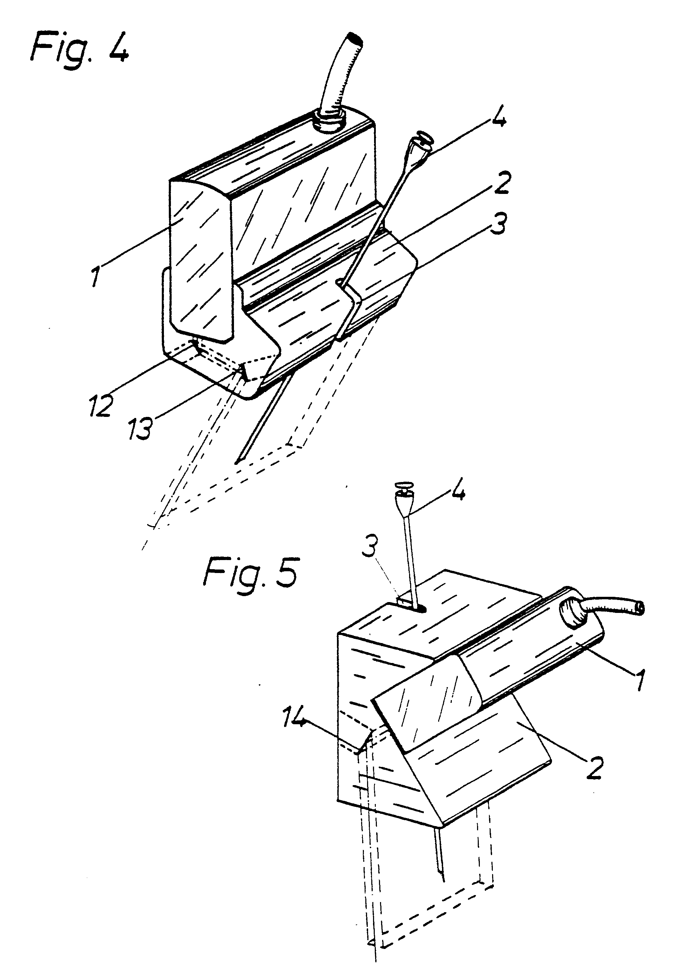

In Figs. 4 and 5 stand-off cells 2 are shown wherein the sound area is reflected once or a number of times on the reflection surfaces 12, 13 and 14. Such reflection surfaces are easy to manufacture as they may consist of the surfaces of air chambers, openings or metal surfaces. The difference in acoustic impedance will thus ensure total reflection. It thus becomes possible to manufacture special stand-off cells for places that are difficult to reach or where accuracy is particularly important. It also becomes possible to give the area a favourable direction and to let the needle 4 follow this.

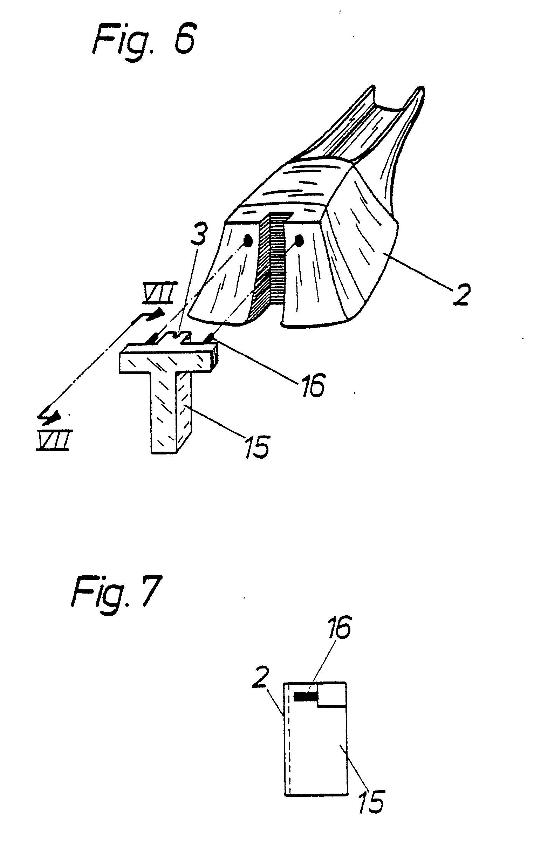

Figs. 6 and 7 show how the needle can be lead in a needle-guide member 15, which is removably secured to the stand-off cell 2 by means of pegs 16.

The stand-off cell according to the invention may be made in a number of different ways according to the intended usage of the stand-off cell. The stand-off cell can also easily be adjusted to any scanner head. Thus, it will be possible for a hospital ward to own a large number of scanner heads so that all biopsy operations may be performed quickly and precisely and with a minimum of discomfort and risk to the patient.

Finally, it should be mentioned that it is also possible to make use of the fact that the ultrasonic waves change direction — are refracted — at the transition between the stand-off cell and the patient due to the difference in acoustic impedance between the stand-off cell and the tissue. This refraction can be used in such a way that the transducer itself can form an angle which differs by 90° from the patient's skin, even when one wishes the ultrasonic waves to penetrate fairly perpendicularly into the patient.

In this way, it becomes possible to lead a hypodermic needle into the patient precisely in the centre of the sound area without the needle having to pass through the transducer itself and thus disturb the images.

## Claims

1. Stand-off cell (2) for an ultrasonic scanner head (1), which stand-off cell can be mechanically mounted to the scanner head (1) and acoustically connected to the emitting surface of the scanner head in a removable manner, and is contactable with the patient through a contact surface (7) thereof, and which furthermore has a number of free surfaces (9), characterized in that the stand-off cell has a slit (3) for engaging a biopsy needle (4) or a hypodermic needle and that the slit (3) extends between that part of the contact surface (7) in the field of view of the scanner head (1) and one of its free surfaces (9), the stand-off cell being made of a material with substantially the same acoustic impedance as that of the patient's tissue.

2. Stand-off cell according to claim 1, characterized in that the contact surface (7) is parallel to the face of attachment (5) of the stand-off cell (2) to the scanner head (1).

3. Stand-off cell according to claims 1-2, characterized in that the stand-off cell (2) has at least one reflection surface (11, 12, 13, 14) for ultrasonic waves.

4. Stand-off cell according to claim 3, characterized in that the contact surface (7) extends at right angles to the face of attachment (5) of the stand-off cell (2) to the scanner head.

5. Stand-off cell according to claims 1-4, characterized in that means (10) are mounted at the slit (3) to guide the needle (4).

6. Stand-off cell according to claim 5, characterized in that the needle-guide means (10) are mounted in such a manner on the stand-off cell (2) that they can be disconnected therefrom and/or are adjustable.

7. Stand-off cell according to claims 1-6, characterized in that the slit (3) is formed in a needle-guide member (15) which is mounted on the stand-off cell (2) in such a manner that it can be disconnected therefrom.

8. Stand-off cell according to one or more of the preceding claims, characterized in that it is made so that the refraction at the surface between the stand-off cell and the patient is used for controlled refraction of the ultrasonic waves.

## Patentansprüche

1. Halterung (2) für einen Ultraschallabtaster (1), welche Halterung am Abtaster (1) mechanisch montierbar und auf lösbare Weise mit der Sendefläche des Abtasters akustisch verbindbar ist, und über eine Berührungsfläche (7) davon mit dem Patienten in Berührung bringbar ist, und die ausserdem eine Anzahl von freien Flächen (9) aufweist, dadurch gekennzeichnet, dass die Halterung einen Schlitz (3) zum Einführen einer Biopsie-Nadel (4) oder einer hypodermischen Nadel aufweist, und dass sich der Schlitz (3) zwischen demjenigen Teil der Berührungsfläche (7), der im Sichtbereich des Abtasters (1) liegt, und einer der freien Flächen (9) erstreckt, wobei die Halterung aus einem Material hergestellt ist, das im wesentlichen die gleiche akustische Impedanz aufweist wie das Gewebe des Patienten.

2. Halterung nach Anspruch 1, dadurch gekennzeichnet, dass die Berührungsfläche (7) parallel zur Befestigungsfläche (5) der Halterung (2) auf den Abtaster (1) ist.

3. Halterung nach dem Ansprüchen 1 oder 2, dadurch gekennzeichnet, dass die Halterung (2) mindestens eine Spiegelungsfläche (11, 12, 13, 14) für Ultraschallwellen aufweist.

4. Halterung nach Anspruch 3, dadurch gekennzeichnet, dass sich die Berührungsfläche (7) rechtwinklig zur Befestigungsfläche (5) der Halterung (2) auf den Abtaster (1) erstreckt.

5. Halterung nach dem Ansprüchen 1 bis 4, dadurch gekennzeichnet, dass am Schlitz (3) Mittel (10) zum Führen der Nadel (4) montiert sind.

6. Halterung nach Anspruch 5, dadurch gekennzeichnet, dass die Nadelführungsmittel (10) an der Halterung (2) auf solche Weise montiert sind, dass sie davon abtrennbar und/oder einstellbar sind.

7. Halterung nach dem Ansprüchen 1 bis 6, dadurch gekennzeichnet, dass der Schlitz (3) in einem Nadelführungselement (15) geformt ist, welches an der Halterung (2) auf solche Weise montiert ist, dass es davon abtrennbar ist.

8. Halterung nach einem oder mehreren der vorangehenden Ansprüche, gekennzeichnet durch eine derartige Ausbildung, dass die Brechung an der Fläche zwischen der Halterung und dem Patienten zur gesteuerten Brechung der Ultraschallwellen verwendet wird.

## Revendications

1. Cellule à fixation verticale (2) pour une tête (1) de scanner à ultrasons, cette cellule à fixation verticale pouvant être montée mécaniquement de façon amovible sur la tête (1) de scanner et reliée acoustiquement à la surface émissive de la tête de scanner, et pouvant être amenée au contact du patient en une surface de contact (7) de la cellule, et présentant en outre un certain nombre de surfaces libres (9), caractérisée en ce que la cellule à fixation verticale comporte une fente (3) pour l'introduction d'une aiguille de biopsie (4) ou d'une aiguille hypodermique, et que la fente (3) s'étend entre la partie de la surface de contact (7) qui se trouve dans le champ de vision de la tête (1) de scanner et une de ses surfaces libres (9), la cellule à fixation verticale étant faite d'un matériau qui présente essentiellement la même impédance acoustique que le tissu du patient.

2. Cellule à fixation verticale selon la revendication 1, caractérisée en ce que la surface de contact (7) est parallèle à la face de fixation (5) de la cellule à fixation verticale (2) à la tête (1) de scanner.

3. Cellule à fixation verticale selon la revendication 1 ou 2, caractérisée en ce que la cellule à fixation verticale (2) comporte au moins une surface de réflexion (11, 12, 13, 14) pour des ondes ultrasonores.

4. Cellule à fixation verticale selon la revendication 3, caractérisée en ce que la surface de contact (7) s'étend à angle droit de la face de fixation (5) de la cellule à fixation verticale (2) à la tête (1) de scanner.

5. Cellule à fixation verticale selon la revendication 1 à 4, caractérisée en ce que des moyens (10) de guidage de l'aiguille (4) sont montés au voisinage de la fente (3).

6. Cellule à fixation verticale selon la revendication 5, caractérisée en ce que les moyens (10) de guidage de l'aiguille sont montés sur la cellule à fixation verticale (2) de manière telle qu'ils peuvent être détachés de celle-ci et/ou sont ajustables.

7. Cellule à fixation verticale selon la revendication 1 à 6, caractérisée en ce que la fente (3) est formée dans un élément (15) de guidage de l'aiguille qui est monté sur la cellule à fixation verticale (2) de manière telle qu'il peut être détaché de celle-ci.

8. Cellule à fixation verticale selon une ou plusieurs des revendications précédentes, caractérisée en ce qu'elle est réalisée de manière telle que la réfraction à la surface située entre la cellule à fixation verticale et le patient est utilisée pour commander la réfraction des ondes ultrasonores.

Fig. 1

Fig. 2

Fig. 3

Fig. 4

Fig. 5

## Fig. 6

## Fig. 7